# EUROPEAN PATENT APPLICATION

(11) **EP 3 910 609 A1**
(43) Date of publication of application: **17.11.2021**
(21) Application number: 21172810.0
(22) Date of filing: 07.05.2021
(51) Int. Cl.: G07F 17/24, A47K 5/00, A61L 2/00

(54) **PARKING PAYMENT DEVICE**

(30) Priority: 12.05.2020 IT 202000010615
(71) Applicant: Flowbird Italia Srl, 20141 Milano (IT)
(72) Inventor: MAGGIONI, Vezio, Milano (IT)
(74) Representative: Vanosi, Adelio Valeriano

(57) **Abstract**

A parking payment device (1) comprising a frame (1A) and payment means (5, 4, 7, 8) which involve physical interaction with at least the fingers or the hand of a user for the operation thereof, characterised by the fact that the said device comprises an automatic dispenser (2) for a hand sanitising product.
Class: G07B

## Description

### FIELD OF THE INVENTION

The present invention relates to a parking payment device.

### BACKGROUND ART

There are parking payment devices commonly known in the art, such as those commonly referred to as 'parking meters'. These are mainly located in the vicinity of urban parking areas, where parking is allowed subject to advance payment of an hourly fee.

Essentially, a user parks the vehicle in the areas marked out (for example with blue lines) and then goes to the nearest parking meter. The user pays to park for a pre-set length of time and the parking meter issues a slip or receipt as proof of payment.

There are also commonly known parking areas which are delimited by automatic barriers, where payment is made after parking. The user enters the area, either taking a ticket or being recognized by an OCR system that records, among other information, the time the vehicle entered the car park and the vehicle's registration number. The vehicle can only leave the parking area once payment has been made by inserting the ticket received at the entrance or by keying the vehicle registration number into the payment terminals present (also known as automatic payment machines and/or parking meters), where the exact fee is calculated based on the length of time the vehicle was parked.

Following this operation, a receipt is issued that allows the vehicle to leave the parking area, guaranteeing the controlled opening of the exit barrier either as a result of the user inserting the payment ticket received from the automatic payment machine or of the system reading the vehicle registration number and recognising that the parking has been paid for the vehicle.

Known payment devices feature physical interaction with the user, who must use the buttons, enter codes (for example, the PIN of a cash or credit card), enter a vehicle registration number (if required) via a keypad or a touch screen, confirm the various operations by pressing buttons to complete or cancel an action.

However, payment devices are used by many people and this is unhygienic.

### SUMMARY OF THE INVENTION

The object of the present invention is to provide a parking payment device which is improved compared with the prior art.

A further object of the invention is to provide a parking payment device which is more hygienic than conventional ones.

This and other objects are achieved by means of parking payment device according to the technical teachings of the claims annexed hereto.

Advantageously, the parking payment device according to the present invention can be made by adding an additional module to devices already installed on roadsides and/or in existing car parks.

### BRIEF DESCRIPTION OF THE FIGURES

Further features and advantages of the innovation will become clearer in the description of a preferred but not exclusive embodiment of the device, illustrated - by way of a non-limiting example - in the drawings annexed hereto, in which:

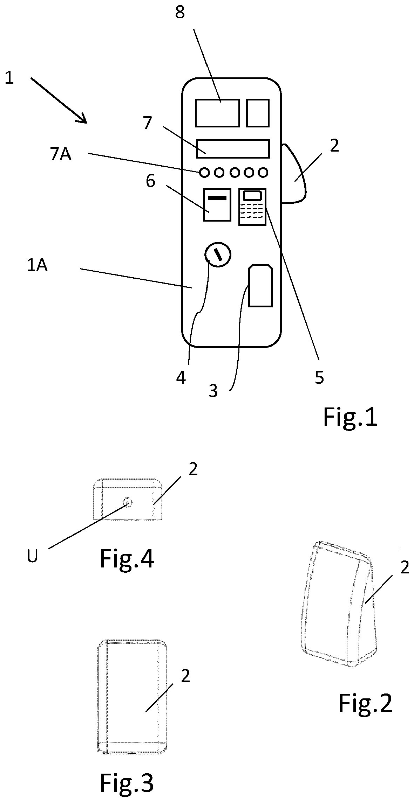

Figure 1 is a simplified, schematic front view of a parking payment device according to the present invention;
Figure 2 is a perspective view of a separate additional module, or of an automatic dispenser for a sanitising product, to be associated with existing devices;
Figure 3 is a front view of the dispenser in Figure 2;
Figure 4 is a bottom-up view of the dispenser in Figure 2;
Figure 5 is a simplified schematic view of the dispenser in Figure 2; and
Figure 6 is a perspective view of the device in Figure 1, in which the dispenser in Figure 2 is clearly visible.

### DETAILED DESCRIPTION OF THE INVENTION

With reference to the figures stated, reference number 1 is used to denote, as a whole, a parking payment device.

The parking payment device 1, which can be a parking meter 1 (also known as a parking payment machine), or an automatic payment machine in a car park with barriers, comprises a frame 1A fastened firmly to the ground, for example on a pavement or curb near paid parking areas (in Italy, these are usually delimited by blue lines, as required by law [i.e. the Italian Street Code]).

After parking the car (or other vehicle) in the parking area, a user goes to the parking meter 1 and proceeds to make the payment for the parking in advance, inserting the amount due, into the coin collection mechanism 4. After inserting the desired amount, the user presses a button 7A and a parking payment slip and/or a receipt is printed (via an appropriate printer 3) stating the 'end' time of the paid parking. Where required, the slip must be displayed on the car dashboard, where it can be checked by a traffic warden.

The parking fees paid are stored in an appropriate manner within device 1, and are collected at regular intervals by employees of the parking area proprietor or parking service provider (local council, transport company, etc.).

Advantageously, the device 1 can also feature a display 8 (possibly of the touch-screen type) for viewing the parking details.

A physical keyboard 7 may also be featured (in addition to buttons 7A) for entering, for example, the vehicle registration number or other relevant information. The keyboard 7 can obviously be integrated into the touchscreen.

The parking payment device 1 can also feature payment means other than the coin collection mechanism 4.

For example, a payment card reader 6 may be featured (Credit: Visa, Mastercard, American Express, Debit: Bancomat, Fastpay, Maestro, Postepay, etc.) including readers for which no contact is required (contactless) and/or with proximity technology (NFC), which may also be coupled with a specific numeric keypad 5 with which to key in the card PIN (where necessary). Obviously, the keypad 5 has specifications for its own operational display unit.

For credit card payments, the parking payment device 1 may be equipped with a network communication terminal, for connection to the internet (or to another proprietary data network), which is designed to connect a machine control unit with the network. Preferably, the communication terminal is a 3G/4G (or in the future 4.5G or 5G) network terminal but it can comprise a network interface via cable or radio (for example WiFi), a data interface via a mobile telephone network, or other known data communication system, as the case may be.

More specifically, when it is necessary to pay for parking, after reading the data on the payment card via the reader 6 (possibly wirelessly) and confirming the payment amount, the control unit sends the data necessary for the payment (via the terminal) to the bank data processor (for example SIA S.p.A.). The bank data processor, using a collection service provider, then credits the amount paid for the parking.

The network connection also allows remote management and total control of the device 1 by means of its own integrated control unit, which manages every operation.

The parking payment device 1 can also be configured to allow payment for other services in addition to parking. For example, it can allow the payment of bus tickets, electricity bills, fines or any other type of service.

Returning to the description of the parking payment device 1, the said device comprises a frame 1A and the aforesaid payment means 5, 4, 7, 8, which involve physical interaction with at least the fingers or the hand of a user for the operation thereof.

According to the invention, the device 1 further comprises an automatic dispenser 2 for a hand sanitising product, the said dispenser being equipped with an interface 10 configured to activate the dispenser for a preset time only once the physical interaction between the payment means 5, 4, 7, 8 and the user has taken place.

Advantageously, the parking payment device 1 comprises a printer 3 for printing a parking payment slip and the interface 10 of the dispenser 2 is configured to detect printer 3 operation and only activate the dispenser 2 once the printing of a slip or receipt has been printed.

The dispenser 2 may feature its own frame 2A, which can be fastened permanently (for example by means of a mechanical coupling with screws/bolts etc.) to the frame 1A of the parking payment device 1.

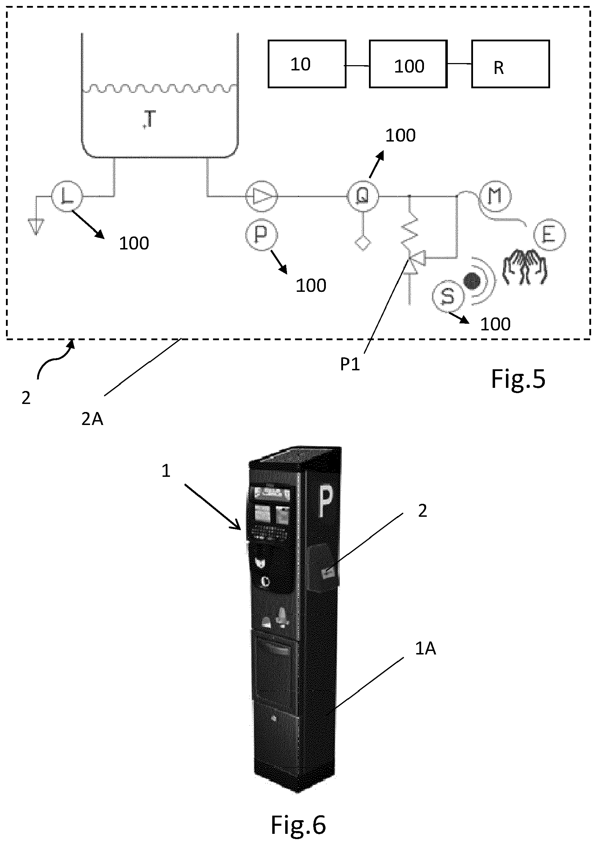

As seen in Figure 5, inside the frame 2A, the dispenser can comprise at least a tank T for a sanitising product, a dispensing nozzle E, and a proximity sensor S configured to activate the dispensing of the sanitising product through the nozzle E only when at least one hand (or another part of the body) of a user is in proximity to said nozzle U.

For dispensing purposes, the dispenser 2 can feature a pump P which pressurises P the fluid contained within the tank T and delivers the said fluid, possibly via a delivery pipe M, to the nozzle E.

Figure 5 shows the tank T containing the disinfectant which can be delivered, by means of a pump P, first to a flow sensor Q and then - via the delivery pipe M and the dispenser E - directly to the user's hands.

The sensor S can detect the presence of the user's hands and emits a pump activation signal, or in any case a delivery signal, via the nozzle U.

A sensor L can measure the level of fluid in the tank while a safety valve P1 can prevent the pressure reaching levels above those considered effective for operation of the instrument.

The dispenser 2 may further comprise a control unit 100 configured to activate the pump P (or in any case delivery via the nozzle U controlled by the sensor S) at the signal from the said interface 10, and which in any case can manage the entire operation of the dispenser 2.

The control unit 100 can therefore be interfaced with all the electronic components of the dispenser.

For example, the control unit 100 can activate the pump and the sensor for a limited or pre-set time (for example, 30 seconds) once the printer has printed the slip or receipt, so that the dispenser 2 remains available and activated for the pre-set time only. In this way, if the user wishes to use the dispenser 2, they can do so immediately after taking the slip or receipt.

Once the pre-set time has lapsed, the dispenser 2 deactivates to prevent wastage or unauthorised activation by people who are not entitled to use it because they have not used the parking payment device (this option is, in any case, entirely at the discretion of the local authority or the service provider which manages the parking areas, and use by the general public can be decided according to public health issues).

It is possible that the control unit 100 is connected to a level sensor L for the liquid present in the tank T and to a radio system R which is interfaced with at least the said printer 3 for printing at least one status or maintenance report for the dispenser 2.

The radio system R may be of a known type, for example Bluetooth, WiFi, etc, and can interface with the parking payment device 1 control unit.

In this way, even if the dispenser 2 is an independent system, it is possible to print a maintenance report (for example after filling the tank, or after replacing it with a pre-filled one) using the remote connection systems already featured on the device 1.

It is also possible that the dispenser 2 control unit 100 communicates directly with the payment device 1 control unit (again, for example, through the radio system R), so as to exploit the 'network' of the device 1 to send status and maintenance reports to an operational control centre for device 1. It is therefore possible to remotely monitor, for example, correct dispenser 2 operation and the necessary filling actions and the effectiveness of the disinfection functions.

Advantageously, therefore, the dispenser may be an independent and essentially autonomous unit, powered by at least one battery and/or by a solar panel.

In this way, the dispenser 2 can simply be 'added onto' or fastened to the parking payment devices already present in the area, thus making these systems safer and more hygienic for a user, in a quick and economical manner.

Essentially, device 2 is comparable to a personal protective equipment system based on the contactless dispensing of a hand sanitising product, provided to support the normal operation (consisting of the issue of payment receipts by parking meters), which can be installed (including therein as an add-on unit) to protect users of paid parking areas by providing the hand disinfection solution for use after contact with the terminals (parking meters) whose surfaces are touched by multiple users.

Activation by means of a proximity sensor S eliminates the need for further contact with other potentially contaminated surfaces.

As already mentioned, device 2 only enables activation of the dispensing of the sanitising/disinfectant product following issuance of a slip (detected via interface 10) and for a limited number of 'dispensing units' (for example, two sprays), as well as for a predetermined length of time within which the user can decide whether or not to use this service (a length of time that can be pre-set - e.g. 30 seconds from the systemic activation thereof), thereby guaranteeing maximum eco-sustainability of the entire system, maximum use, and minimum environmental impact in the application of the function.

The interface 10, which can detect activation of the printer, may comprise an electronic device which remotely detects variations in the magnetic field generated by the printer of the parking payment device and, thanks to its own expert system, determines whether they are produced exclusively by the activation device (the printer), thereby allowing them to be distinguished from those of any external stimulus to prevent false activation or fraudulent activation.

Optionally, the dispenser 2 may be controlled (for example by means of the radio system R, or other similar device, for example IR, serial cable, etc.) also for routine maintenance operations when necessary and for topping up the disinfectant liquids when necessary. It is also possible that programming (of the control unit 100) may be allowed in order to set the duration and the number of dispensing units allowed, including therein through the use of a specific maintenance app (software) installed on terminals (mobile phones, etc.) in use by maintenance engineers.

The control unit 100 can record (for example in an integrated memory) a dispensing log and use the latter to improve the operation thereof; furthermore, in the event that alarms are activated, it communicates this directly to the management application (app) on the mobile terminal.

As already mentioned, the dispenser 2 may be an autonomous device, for external use, which does not require a power supply since it is battery-operated.

Alternatively, the dispenser 2 may be fully integrated into the parking payment device 1, and all its components may be physically connected and controlled (as well as powered) by the device 1 or the control unit therefor.

In this case, the interface 10 is a physical interface, which can comprise cables, quick connectors, etc. Therefore, the control unit for the device 1 can activate the dispenser 2 when appropriate, and control all the components thereof.

As already mentioned, the tank T can be independent, and therefore constitute a consumable product, equipped with appropriate quick release fasteners for the quick replacement thereof during maintenance.

In any case, the tank T can contain disinfectant products (for example liquid, cream, foam products etc.) which are specifically formulated for hand disinfection, in compliance with environmental legislation in force.

Advantageously, during use, the pump P can emit (in cooperation with the nozzle U) a calibrated dose of disinfectant at each dispensing action.

Therefore, dispensing can only take place if the dispenser 2 is active and solely following issue of a slip or receipt due to the positioning of the hand under the dispenser (i.e. near the sensor S).

The control unit 100 can monitor level, flow, and pressure data to carry out the entire series of measurements needed to ensure the instrument operates as designed.

In addition, the control unit 100 can include software that allows you to view the operating data, all of which can be controlled (as already reported) directly through the dedicated app.

If necessary, the distributor can include additional options, for example, for the dispensing of bags for the disposal of dog waste (by means of an additional module integrated into the dispenser 2).

In the text above, a dispenser 2 is described which can be integrated into a new payment device, or which forms an independent unit to be simply added to existing devices 1 already installed.

The timed activation of the dispenser 2 (subject to activation of the sensor S) has also been linked to the activation of a printer 3 of the device 1. This is because the printer is a part whose operation is easily detectable, including therein remotely, by means of suitable sensors, such as the interface 10.

It should be noted that the interface 10 can be controlled in such a way that the dispenser is also activated independently of the activation of the printer. This can be done, for example, through the aforesaid radio means R, which communicate directly with the device 1 control unit.

In this case, the dispenser 2 may therefore be positioned on its own frame or column independently of the device 1, but close thereto or in the immediate vicinity thereof. In the present text, the term 'in the immediate vicinity thereof' or 'close thereto' means that the dispenser 2 is immediately accessible via device 1, after use thereof (for example, positioned between 0 m and 1.5 m away from device 1).

Hypothetically, therefore, the integrated control unit of the device 1 can be programmed to activate the dispenser even when the touchscreen 8, the keyboard 7, the buttons 7A, or another part of the machine is touched. This also applies, of course, in the event of an 'attempted' payment procedure.

It is also possible that the activation of the dispenser 2 may be an additional service, provided at a charge, in addition to those already featured and enabled by device 1.

For example, therefore, in the device 1 control menu, a sanitation service can also be provided for a pre-set price.

For example, if a user wishes to sanitise their hands, regardless of whether or not they have made a parking payment, they can insert a pre-set amount (for example 10 or 20 cents, or just a symbolic amount), and after this the device can activate the dispenser 2. In this context, the activation of the dispenser can take place upon printing a receipt for the hand sanitisation operation, or upon receipt of a direct command from the device 1 control unit. This depends on the 'type' of the interface 10.

Various embodiments of the innovation have been disclosed herein, but further embodiments may also be conceived using the same innovative concept.

## Claims

1. A parking payment device (1) comprising a frame (1A) and payment means (5, 4, 7, 8) needing a physical interaction with at least the fingers or hand of a user for their actuation, **characterized in that** it comprises an automatic dispenser (2) for a hand sanitizing product, the automatic dispenser (2) being equipped with an interface (10) which activates it for a predetermined time, only after the physical interaction between the payment means (5, 4, 7, 8) and the user has taken place, the parking payment device (1) comprising a printer (3) for printing a parking ticket or a receipt, and the interface (10) of the automatic dispenser (2) being configured to detect the activation of the printer (3) as activation signal of the distributor (2).

2. Device (1) according to the previous claim wherein the distributor (2) is fixed externally to the frame (1A) of the device (1), the distributor (2) optionally being an independent unit powered by at least one battery and/or via a solar panel.

3. Device according to claim 1, wherein the distributor (2) provides at least one tank (T) of sanitizing product, a dispensing nozzle (E), a proximity sensor (S) configured to activate the dispensing of the sanitizing product through the nozzle (E) only when at least one hand of a user is in proximity of said nozzle.

4. Device according to the previous claim, wherein the distributor comprises a pump (P) that pressurizes (P) the fluid contained inside the tank (T) and sends it to the nozzle (E).

5. Device according to the preceding claim, wherein the distributor comprises a control unit (100) configured to activate the pump (P) on the signal of said interface (10).

6. Device according to the preceding claim wherein the control unit (100) is connected to a level sensor (L) and to a radio system (R) interfaced at least with said printer (3) for printing at least one report of distributor maintenance (2).

7. Device according to claim 1, wherein the activation of the device (2) takes place upon emission of a receipt of payment of a rate dedicated to hand hygiene.
